# EUROPÄISCHE PATENTANMELDUNG

(11) **EP 2 375 238 A1**
(43) Veröffentlichungstag der Anmeldung: **12.10.2011**
(21) Anmeldenummer: 10193198.8
(22) Anmeldetag: 30.11.2010
(51) Int. Cl.: G01N 21/05, G01N 21/85, G01N 33/18, B01L 3/00, B01D 19/00

(54) **Flüssigkeitsanalysegerät**

(30) Priorität: 01.04.2010 WO PCT/EP2010/054402
(71) Anmelder: Hach Lange GmbH, 14163 Berlin (DE)
(72) Erfinder: Uthemann, Rolf, 51373, Leverkusen (DE); Dr.de Heij, Bas, 41542, Dormagen (DE); Berggold, Kai, 50823, Köln (DE); Dr. Farjam, Aria, 40223, Düsseldorf (DE); Dr. Lundgreen, Ulrich, 33330, Gütersloh (DE); Dr. Hahn, Markus, 47906, Kempen (DE)
(74) Vertreter: Ter Smitten, Hans

(57) **Zusammenfassung**

Die Erfindung bezieht sich auf einen Flüssigkeitsanalysegerät (10) zur fotometrischen Bestimmung eines Analyts in einer Flüssigkeit. Das Flüssigkeitsanalysegerät weist ein stationäres Basismodul (12) auf, das ein Fotometer (50) ohne Messstrecke (68) und keine flüssigkeitsführenden Leitungen aufweist. Erste Flüssigkeitsanalysegerät (10) weist ferner ein austauschbares Fluidikmodul (14) auf, das alle flüssigkeitsführenden Leitungen mit einem die Messstrecke (68) bildenden Messkanal (32) und eine Entgasungsvorrichtung (70) zum Entgasen der Flüssigkeit aufweist.

Die Entgasungsvorrichtung (70) weist einen den Messkanal (32) radial umschließenden Frittenkörper (90) auf, der an eine Unterdruckquelle (76) angeschlossen ist.

## Beschreibung

Die Erfindung bezieht sich auf ein Flüssigkeitsanalysegerät zur fotometrischen Bestimmung eines Analyts in einer Flüssigkeit, und bezieht sich insbesondere auf ein quasi-kontinuierlich messendes Prozess-Flüssigkeitsanalysegerät zur Bestimmung eines Analyts in Wasser, insbesondere in Abwasser.

Flüssigkeitsanalysegeräte werden beispielsweise zur Überwachung eines Analyts in Abwasser oder Trinkwasser eingesetzt, um die AbwasserAufbereitung zu regeln beziehungsweise die Trinkwasser-Qualität zu kontrollieren. Insbesondere für die über einen langen Zeitraum quasi-kontinuierlich messenden Prozess-Flüssigkeitsanalysegeräte haben sich inzwischen modular aufgebaute Flüssigkeitsanalysegeräte bewährt, wie sie beispielsweise aus EP 0 706 659 B1 bekannt sind. Hierbei sind ein Teil der Fluidik und eine Dialysemembran in einem austauschbaren Fluidikmodul angeordnet, wohingegen die Pumpen und die Vorratsbehälter für die Trägerflüssigkeit und das Reagenz in einem Basismodul vorgesehen sind.

In der Flüssigkeitsprobe können Gasblasen dadurch entstehen, dass sich die Probe in dem Fluidikmodul erwärmt, beispielsweise durch eine an der Dialysemembran anliegende warme Analyseflüssigkeit. Ferner kann ein saures Reagenz in der Flüssigkeitsprobe Kohlendioxyd-Gas austreiben. Bei der Fotometrie können schon wenige kleine Gasblasen in der Flüssigkeitsprobe zu völlig falschen Messergebnissen führen, was in besonders starkem Maße für mikrofluidische Strukturen gilt, wie sie wegen ihres geringen Reagenzienverbrauchs für über einen langen Zeitraum quasi-kontinuierlich messende Prozess-Flüssigkeitsanalysegeräte besonders interessant sind.

Aufgabe der Erfindung ist es demgegenüber, ein modular aufgebautes fotometrisches Flüssigkeitsanalysegerät mit einer effektiven Entgasungsvorrichtung zu schaffen.

Diese Aufgabe wird erfindungsgemäß gelöst mit einem Flüssigkeitsanalysegerät mit den Merkmalen des Patentanspruches 1.

Das Flüssigkeitsanalysegerät weist ein stationäres Basismodul auf, das ein Fotometer ohne Messstrecke und keine flüssigkeitsführenden Leitungen aufweist. Das Basismodul ist also frei von jeder Flüssigkeits-Fluidik. Ferner ist ein austauschbares Fluidikmodul vorgesehen, das die gesamte Flüssigkeits-Fluidik, also alle flüssigkeitsführenden Leitungen einschließlich eines die Messstrecke für das Fotometer bildenden Messkanals aufweist.

Das Fluidikmodul weist eine Entgasungsvorrichtung auf, die von einem den Messkanal radial umschließenden Frittenkörper gebildet wird, der an eine Unterdruckquelle, beispielsweise an eine Pumpe oder an einen Unterdruckspeicher, angeschlossen ist. Durch die Entgasungsvorrichtung wird also unmittelbar die sich in dem Messkanal befindliche Probenflüssigkeit entgast. Der Frittenkörper umgibt den Messkanal in Umfangsrichtung mindestens annähernd vollständig, so dass der Messkanal über annähernd seinen gesamten Außenumfang und annähernd seine gesamte Länge entgast wird.

Der Messkanal dient sowohl als Messstrecke für das Fotometer als auch als Entgasungsstrecke. Hierdurch entfällt eine separate Entgasungsstrecke, weshalb das Gesamtvolumen der Flüssigkeits-Leitungen in dem Fluidikmodul entsprechend klein ausfallen kann. Hierdurch wiederum verkürzen sich die Transportwege in dem Fluidikmodul und verringert sich die Menge an Spülflüssigkeit, die zum Spülen der Flüssigkeits-Leitungen nach Abschluss eines Messzyklus erforderlich ist. Dies ist insbesondere bei einem Prozess-Flüssigkeitsanalysegerät von Bedeutung, da die bevorratbare Menge an Spülflüssigkeit begrenzt ist. Durch die großflächige Entgasung kann die Länge des durch den Messkanal gebildeten Entgasungskanals so klein ausfallen, wie sie für die Fotometrie gerade noch ausreichend ist.

Im Messbetrieb des Flüssigkeitsanalysegerätes wird in einer Probengewinnungsvorrichtung zunächst eine Flüssigkeitsprobe gewonnen, beispielsweise durch Dialyse. Der Flüssigkeitsprobe wird ein Reagenz zugeführt, und diese Flüssigkeitsprobe in den Messkanal gepumpt. Während der Dauer der Reaktionszeit, die typischerweise 1-10 min beträgt, ist die Unterdruckquelle aktiv, so dass über den Frittenkörper das in der Flüssigkeitsprobe befindliche Gas über die Dauer der Reaktionszeit abgepumpt wird. Der von der Unterdruckquelle typischerweise generierte absolute durchschnittliche Druck beträgt dabei 0,1 bis 0,7 bar. Für die anschließende Fotometrie wird die Unterdruckquelle bevorzugt wieder abgeschaltet, um für die Fotometrie möglichst konstante optische Verhältnisse zu erhalten.

Der Frittenkörper besteht bevorzugt aus einem porösen und gasdurchlässigen Material, das hydrophob ist. Vorzugsweise besteht der Frittenkörper aus PTFE, also aus Polytetrafluoräthylen. PTFE weist eine geeignete Porengröße und eine hohe Hydrophobie auf, so dass die Entgasung mit einem relativ hohen Differenzdruck erfolgen kann, ohne dass Flüssigkeit in den Frittenkörper eindringen kann. Durch den relativ hohen Differenzdruck wird eine ausreichend schnelle Entgasung sicherstellt, die nicht länger dauern muss als die Dauer der Reaktion des Reagenzes mit dem Analyt in der Wasserprobe. Hierdurch wiederum werden relativ kurze Messezyklen realisiert, die für eine engmaschige quasi-kontinuierliche quantitative Bestimmung des Analyts der Flüssigkeitsprobe wichtig ist. Eine möglichst engmaschige Analyt-Bestimmung ist wiederum für die Regelung eines Prozesses, beilspielsweise eines Klärprozesses in einer Kläranlage von großer Wichtigkeit.

Gemäß einer bevorzugten Ausgestaltung ist der Frittenkörper zylindrisch ausgebildet, und zwar sowohl innenseitig als auch außenseitig, und bildet auf diese Weise einen Hohlzylinder. Hierdurch wird eine homogene Entgasung über die gesamte Länge und den gesamten Umfang des Messkanals sichergestellt, so dass die Fotometrie mit hoher Genauigkeit und geringer Fehlerhäufigkeit durchgeführt werden kann.

Vorzugsweise ist die Unterdruckquelle in dem Basismodul angeordnet. Wenn in dem Basismodul ohnehin eine Unterdruckquelle, also beispielsweise ein Unterdruckspeicher oder eine pneumatische Pumpe vorgesehen ist, die beispielsweise zum Antrieb peristaltischer Pumpenmimiken des Fluidikmoduls dient, kann diese Unterdruckquelle auch als Unterdruckquelle zur Erzeugung eines entsprechenden Unterdrucks in dem Frittenkörper benutzt werden. Eine separate Unterdruckquelle ist daher nicht erforderlich, so dass der konstruktive Aufwand für die Unterdruckquelle sehr gering ausfällt.

Gemäß einer bevorzugten Ausgestaltung weist der Frittenkörper außenseitig eine radiale Ausnehmung auf, die mit einer an die Unterdruckquelle angeschlossenen Entgasungsleitung verbunden ist. Der Frittenkörper wird üblicherweise in einen Fluidikkörper des Fluidikmoduls durch Umspritzen oder durch Einkleben mit einem Klebemittel beziehungsweise mit einem Lösungsmittel flüssigkeitsdicht integriert. Hierbei kann die äußere Grenzschicht des Frittenkörpers derart verändert werden, dass die Grenzschicht nicht mehr gasdurchlässig ist. Daher wird nach dem Umspritzen beziehungsweise dem Einkleben des Frittenkörpers in den Fluidikkörper von außen in die Frittenkörper-Grenzschicht eine entsprechende Ausnehmung eingebracht, beispielsweise gebohrt oder gefräst.

Vorzugsweise weist das Fluidikmodul einen flachen Kunststoff-Fluidikkörper auf, in dem die Flüssigkeits-Leitungen obenseitig und/oder untenseitig eingelassen sind. Der Messkanal steht senkrecht zur Grundebene des Fluidikkörpers, erstreckt sich also maximal nur über die Dicke des Fluidikkörpers. Durch eine derartige Anordnung wird die Fertigung erheblich vereinfacht, da der Frittenkörper senkrecht zur Grundebene des Fluidikkörpers in eine entsprechende Bohrung in dem Fluidikkörper eingesetzt und fixiert werden kann, beispielsweise durch Umspritzen, Einkleben Einschweißen, Laserschweißen etc.

Vorzugsweise sind die beiden Längsenden des Frittenkörpers jeweils mit einem fotometrisch transparenten und flüssigkeitsdichten Fensterkörper verschlossen. Der Fensterkörper kann beispielsweise jeweils von einer transparenten Kunststoff-Folie gebildet werden, die flüssigkeitsdicht auf die beiden Längsenden des Frittenkörpers aufgebracht ist, beilspielsweise durch Verkleben oder Verschweißen.

Gemäß einer bevorzugten Ausgestaltung ist an den beiden Stirnseiten des Frittenkörpers jeweils eine Radialnut als Messkanal-Einlassleitung beziehungsweise -Auslassleitung vorgesehen. Auf diese Weise wird mit einfachen Mitteln, nämlich in Form der beiden radialen Nuten, die Einlassleitung und die Auslassleitung für den Messkanal geschaffen.

Im folgenden wird unter Bezugnahme auf die Zeichnungen ein Ausführungsbeispiel der Erfindung näher erläutert.

Es zeigen:
- Figur 1: eine schematische Darstellung eines ProzessFlüssigkeitsanalysegerätes, bestehend aus einem Basismodul und einem austauschbaren Fluidikmodul mit einer Entgasungsvorrichtung,
- Figur 2: einen Längsschnitt durch ein Flüssigkeitsanalysegerät gemäß der Fig. 1,
- Figur 3: eine Draufsicht auf das Fluidikmodul des Flüssigkeitsanalysegerätes der Figur 2, und
- Figur 4: ein Längsschnitt durch das Fluidikmodul des Flüssigkeitsanalysegerätes der Figur2.

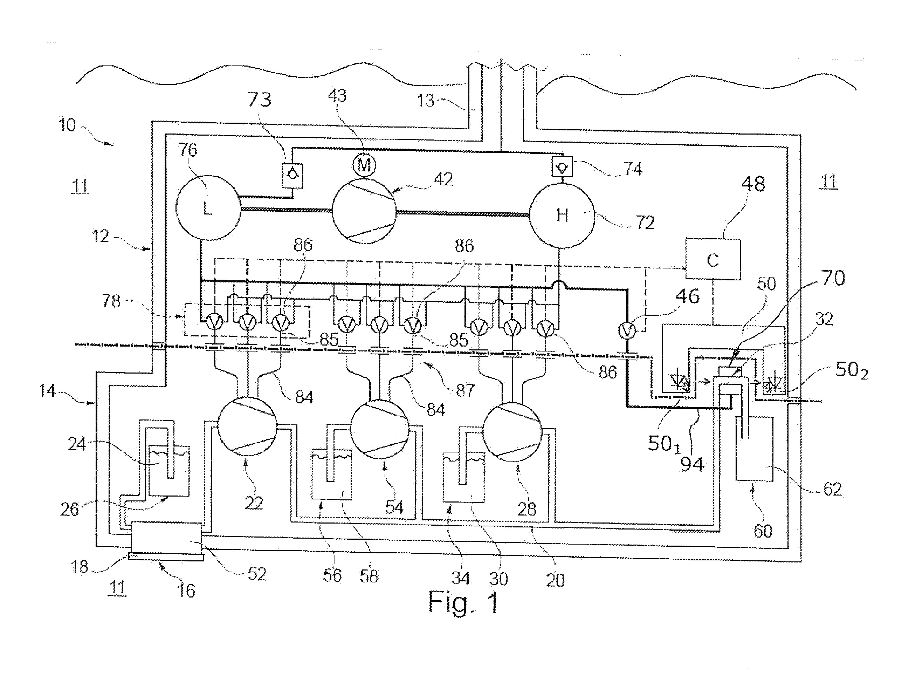

In der Figur 1 ist schematisch ein Prozess-Flüssigkeitsanalysegerät 10 zur kontinuierlichen beziehungsweise quasi-kontinuierlichen quantitativen fotometrischen Bestimmung eines Analyten, beispielsweise Phosphat, Ammonium oder Nitrat, in einer Analyseflüssigkeit 11, beispielsweise Abwasser, dargestellt. Das Flüssigkeitsanalysegerät 10 ist vorliegend ein stationäres Flüssigkeitsanalysegerät 10 und ist in die wässrige Analyseflüssigkeit 11 eingetaucht montiert, ist also als sogenannte Tauchsonde ausgebildet. Das Flüssigkeitsanalysegerät 10 besteht aus einem Basismodul 12, das an einem Bohrgestänge 13 starr in der Analyseflüssigkeit 11 oder knapp darüber aufgehängt ist, und einem austauschbaren Fluidikmodul 14, das an dem Basismodul 12 lösbar befestigt und in die Analyseflüssigkeit 11 eingetaucht ist. Das Fluidikmodul 14 kann bei Bedarf gegen ein neues Fluidikmodul ausgetauscht werden.

Die gesamte Flüssigkeits-Fluidik des Flüssigkeitsanalysegerätes 10 ist in dem Fluidikmodul 14 vorgesehen. Das Fluidikmodul 14 weist einen Trägerflüssigkeits-Vorratstank 26 mit einer Trägerflüssigkeit 24 auf, die über eine Leitung an eine Probengewinnungsvorrichtung 16 angeschlossen ist, die vorliegend eine Dialysevorrichtung 16 ist. Die Dialysevorrichtung 16 weist eine Dialysemembran 18 auf, die den Dialyseraum 52 von der Analyseflüssigkeit 11 trennt, in dem die Trägerflüssigkeit während der Dialyse verweilt. Der Dialyseraum 52 kann beispielsweise von einer mäanderartig verlaufenden Nut 37 gebildet werden, deren Nutöffnung durch die Dialysemembran 18 verschlossen ist. Hinter der Dialysevorrichtung 16 ist eine erste Pumpmimik 22 vorgesehen, die die Flüssigkeitsprobe bzw. das Dialysat aus der Dialysevorrichtung 16 zu einem Messkanal 32 pumpt.

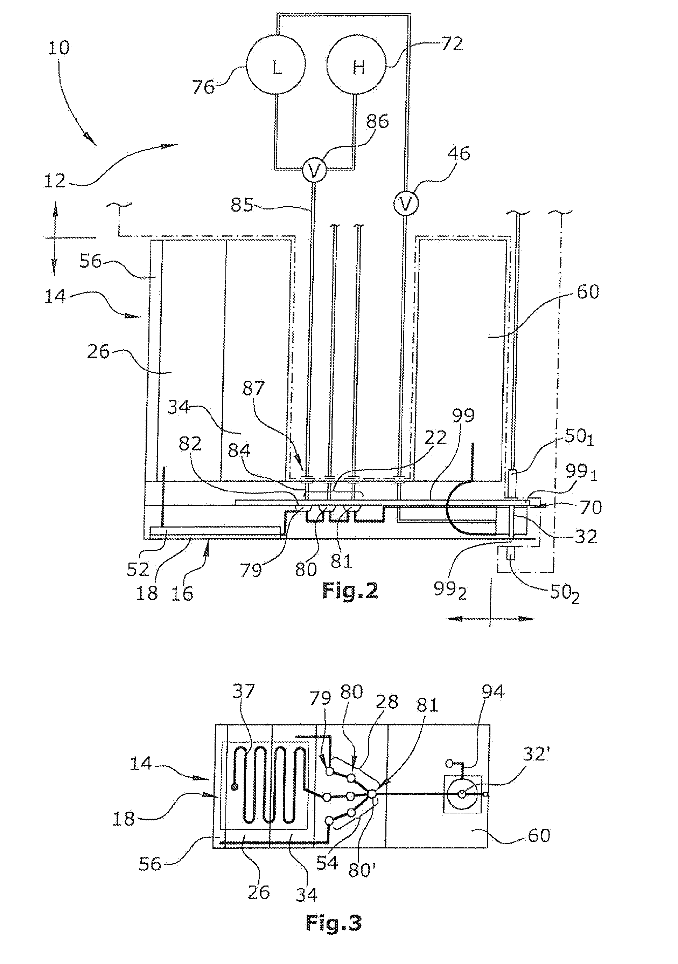

Das Fluidikmodul 14 weist einen Reagenz-Vorratstank 34 mit einem flüssigen Reagenz 30 auf, das durch eine zweite Pumpmimik 28 zu dem Messkanal 32 gepumpt wird. Ferner ist in dem Fluidikmodul 14 ein Standardlösungs-Vorratstank 56 mit einer Standardlösung 58 vorgesehen, wobei eine dritte Pumpmimik 54 vorgesehen ist, die die Standardlösung bei Bedarf zu dem Messkanal 32 pumpt. Die drei Pumpmimiken 22, 28, 54 laufen sternförmig zusammen, wie insbesondere in Figur 3 gut zu erkennen ist.

Die Flüssigkeit beziehungsweise die Flüssigkeitsprobe fließt von den drei Pumpmimiken 22,28,54 zu einer Fotometer-Messstrecke 68, und von dort aus in einen Abfallflüssigkeits-Tank 60, in dem die Abfallflüssigkeit 62 gesammelt wird. Die Fotometer-Messstrecke 68 ist funktional einem basismodulseitigen Fotometer 50 zugeordnet, das eine Lichtquelle 50₁ und einen Empfänger 50₂ aufweist, zwischen denen ein die Fotometer-Messstrecke 68 bildender Messkanal 32 der Flüssigkeitsleitung angeordnet ist. Das Fotometer 50 ist vorliegend als Transmissions-Fotometer ausgebildet. Das Fotometer 50 kann alternativ jedoch auch als Reflektions-Fotometer ausgebildet sein.

Als Druckquellen zum Antrieb der drei Pumpmimiken 22,54,28 dienen ein Überdruck-Speicher 72 und ein Unterdruck-Speicher 76 in dem Basismodul 12. Der Unterdruck-Speicher 76 bildet eine Unterdruckquelle 76. Die drei Pumpmimiken 22,54,28 sind als pneumatische Peristaltikpumpen ausgebildet. Jeder Pumpmimik 22,54,28 ist jeweils eine Pumpen-Aktuatorik 78 zugeordnet, die jeweils aus drei Umschaltventilen 86 besteht. Jede Pumpmimik 22,54,28 weist jeweils drei Pumpkammern 79,80,81 mit jeweils einer elastischen Pumpenmembran 82 auf, die aus Gummi oder aus einem elastischen Kunststoff besteht.

Die Rückseite der Pumpenmembran 82 ist jeweils über eine fluidikmodulseitige pneumatische Steuerleitung 84, eine Steuerleitungs-Kupplung 87 und eine basismodulseitige pneumatische Steuerleitung 85 mit einem Umschaltventil 86 verbunden, das die Pumpenmembran 82 wahlweise mit dem Überdruck-Speicher 72 oder dem Unterdruck-Speicher 76 verbindet. Auf diese Weise wird die Rückseite der Pumpenmembran 82 entweder mit Überdruck oder mit Unterdruck beaufschlagt, so dass die Pumpkammern 79,80,81 gefüllt oder geleert werden. Durch sukzessives Füllen und Leeren der drei Pumpkammern einer der Pumpmimiken 22,54,28 wird eine peristaltische Pumpbewegung generiert.

Zur Erzeugung des Unterdrucks in dem Unterdruck-Speicher 76 und des Überdrucks in dem Überdruck-Speicher 72 ist eine Pneumatikpumpe 42 in dem Basismodul 12 vorgesehen, deren Pumpeneinlass mit dem Unterdruck-Speicher 76 und deren Pumpenauslass mit dem Überdruck-Speicher 72 verbunden ist. Die Pneumatikpumpe 42 wird ständig durch einen elektrischen Pneumatikpumpen-Motor 43 angetrieben. Der Unterdruck in dem Unterdruck-Speicher 76 und der Überdruck in dem Überdruck-Speicher 72 wird jeweils durch ein entsprechendes Unterdruckventil 73 beziehungsweise Überdruckventil 74 begrenzt, die jeweils mit atmosphärischem Luftdruck verbunden sind. Alternativ können in den Speichern 72, 76 auch Drucksensoren vorgesehen sein, durch die bei Über- bzw. Unterschreiten eines Grenzdruckes die Pneumatikpumpe ein- bzw. ausgeschaltet wird. Das den Unterdruck in der Entgasungsvorrichtung 70 steuernde Entgasungs-Ventil 46 ist an den Unterdruck-Speicher 76 angeschlossen.

Wie insbesondere in den Figuren 2 und 3 erkennbar ist, weist das Fluidikmodul 14 einen plattenförmigen Kunststoff-Fluidikkörper 98 auf, der die Flüssigkeits-Fluidikleitungen, die Pumpkammern 79,80,81, die Dialysevorrichtung 16, sowie den Messkanal 32 mit der Entgasungsvorrichtung 70 aufweist. Ferner weist das Fluidikmodul 14 die Tanks 26,56,34,60 auf, die auf den plattenförmigen Kunststoff-Fluidikkörper 98 aufgesetzt sind.

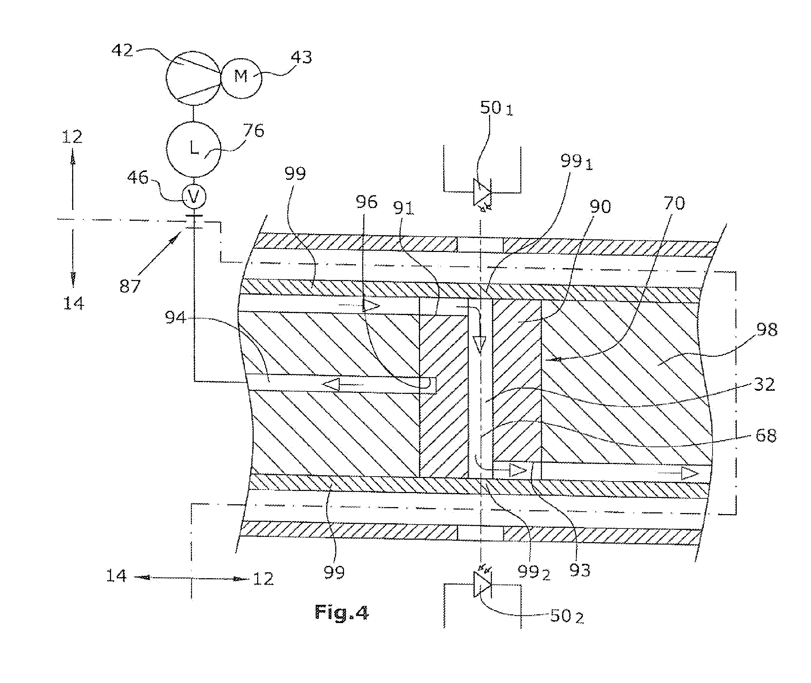

Wie insbesondere in der Figur 4 dargestellt ist, weist die Entgasungsvorrichtung 70 einen hohlzylindrischen Frittenkörper 90 aus PTFE (Handelsname "Teflon") auf, dessen Hohlraum den Messkanal 32 bildet, der die Messstrecke 68 für das Fotometer 50 darstellt. Der Frittenkörper 90 kann alternativ aus jedem geeigneten Kunststoff bestehen, der einen Durchtrittsdruck von 1,5 bar gewährleistet, was bei Kunststoffen gewährleistet ist, die einen Kontaktwinkel (mit Wasser) von mindestens 85°-90° aufweisen, Dies ist beispielsweise für PE (Polyethylen) der Fall.

Die Axiale des Frittenkörpers 90 steht senkrecht zu der Grundebene des flachen Fluidikkörpers 98. Die axiale Länge des Frittenkörpers 90 entspricht annähernd der Dicke des Fluidikkörpers 98, und kann zwischen 1,5 und 5 mm liegen. Die radiale Dicke des Frittenkörpers 90 kann zwischen 1,0 und 3,5 mm und der Durchmesser des Messkanals 32 weniger als 1,0 mm betragen, so dass der Außendurchmesser des Frittenkörpers 90 in der Regel zwischen 3 und 7 mm liegt.

Der Frittenkörper 90 ist in den Fluidikkörper 98 eingegossen, kann jedoch auch mit Hilfe von Lösungsmitteln oder einem Klebstoff in einer entsprechenden Öffnung des Fiuidikkörpers 98 fixiert sein. Hierbei wird in aller Regel die äußere zylindrische Oberfläche des Frittenkörpers 90 so verändert, dass diese nicht mehr gasdurchlässig ist. Aus diesem Grund weist der Frittenkörper 90 außenseitig eine radiale Ausnehmung 96 auf, die über die daran angeschlossene Entgasungsleitung 94 an die Unterdruckquelle, nämlich den Unterdruck-Speicher 76 angeschlossen ist.

Der Frittenkörper 90 weist an seinen beiden Stirnseiten jeweils eine Radialnut 91,93 auf, wobei eine Radialnut 91 die Messkanal-Einlassleitung und die andere Radialnut 93 die Messkanal-Auslassleitung bildet, durch die die zu fotometrierende Flüssigkeitsprobe in den Messkanal 32 hinein fließt beziehungsweise aus diesem heraus fließt. Der Fluidikkörper 98 ist in diesem Bereich auf beiden Seiten durch eine fotometrisch Transparente Folie 99 bedeckt, wie beispielsweise durch Laserschweißen entlang der Fluidikkanäle auf geschweißt ist. Die Folie 99 bildet an den beiden Stirnseiten des Frittenkörpers 90 jeweils einen Fensterkörper 99₁,99₂, der den Eintritt und Austritt des Messstrahles des Fotometers 50 erlaubt.

Alle Ventile 86,46 und das Fotometer 50 werden durch eine zentrale Steuerung 48 gesteuert. Alle elektrischen Bauteile sind in dem Basismodul 12 angeordnet.

## Patentansprüche

1. Flüssigkeitsanalysegerät (10) zur fotometrischen Bestimmung eines Analyts in einer Flüssigkeit, mit
einem stationären Basismodul (12), das ein Fotometer (50) ohne Messstrecke (68) und keine flüssigkeitsführenden Leitungen aufweist, und
einem austauschbaren Fluidikmodul (14), das alle flüssigkeitsführenden Leitungen mit einem die Messstrecke (68) bildenden Messkanal (32) und eine Entgasungsvorrichtung (70) zum Entgasen der Flüssigkeit aufweist,
wobei die Entgasungsvorrichtung (70) einen den Messkanal (32) radial umschließenden Frittenkörper (90) aufweist, der an eine Unterdruckquelle (76) angeschlossen ist.

2. Flüssigkeitsanalysegerät (10) nach Anspruch 1, wobei der Frittenkörper (90) aus PTFE besteht.

3. Flüssigkeitsanalysegerät (10) nach einem der vorangegangenen Ansprüche, wobei der Frittenkörper (90) hohlzylindrisch ausgebildet ist.

4. Flüssigkeitsanalysegerät (10) nach einem der vorangegangenen Ansprüche, wobei die Unterdruckquelle (76) in dem Basismodul (12) angeordnet ist.

5. Flüssigkeitsanalysegerät (10) nach einem der vorangegangenen Ansprüche, wobei der Frittenkörper (90) außenseitig eine radiale Ausnehmung (96) aufweist, die mit einer an die Unterdruckquelle (76) angeschlossenen Entgasungsleitung (94) verbunden ist.

6. Flüssigkeitsanalysegerät (10) nach einem der vorangegangenen Ansprüche, wobei das Fluidikmodul (14) einen flachen Kunststoff-Fluidikkörper (98) aufweist, in dem die Flüssigkeits-Leitungen eingelassen sind, wobei der Messkanal (32) senkrecht zur Grundebene des Fluidikkörpers (98) steht.

7. Flüssigkeitsanalysegerät (10) nach einem der vorangegangenen Ansprüche, wobei die beiden Längsenden des Frittenkörpers (90) jeweils mit einem fotometrisch transparenten Fensterkörper (99₁,99₂) verschlossen sind.

8. Flüssigkeitsanalysegerät (10) nach einem der vorangegangenen Ansprüche, wobei an den beiden Stirnseiten des Frittenkörpers (90) jeweils eine Radialnut (91,93) als Messkanal -Einlassleitung und -Auslassleitung vorgesehen ist.
